# EUROPEAN PATENT APPLICATION

(11) **EP 4 803 090 A1**
(43) Date of publication of application: **09.09.2026**
(21) Application number: 24883707.2
(22) Date of filing: 04.11.2024
(51) Int. Cl.: A61K 35/17, A61P 37/02, C12N 5/0783

(54) **METHOD FOR INDUCING AN ANTI-INFLAMMATORY PHENOTYPE IN T-CELLS USING MESENCHYMAL STEM CELL-DERIVED MITOCHONDRIA**

(30) Priority: 03.11.2023 CL 202303283
(71) Applicant: Cells For Cells S.A., Santiago 7550101 (CL); Universidad de los Andes, 7550000 Santiago (CL)
(72) Inventor: LUZ CRAWFORD, Patricia, Santiago, 7550101 (CL); LUQUE CAMPOS, Noymar, Santiago, 7550101 (CL); KHOURY, Maroun, Santiago, 7550101 (CL)
(74) Representative: Elzaburu S.L.P.
(86) International application number: PCT/CL2024/050138
(87) International publication number: WO 2025/091139

(57) **Abstract**

The inventors have shown that the incorporation of mitochondria derived from mesenchymal stem cells by T cells induces a significant increase in their anti-inflammatory properties. This innovation could allow the reprogramming of T cells isolated from individuals with inflammatory and/or autoimmune diseases, through the acquisition of specific mitochondria (MT) that induce an anti-inflammatory phenotype. The obtained T-MT cells could be applied as therapeutic agents to effectively reduce inflammation, and therefore, the symptoms of these diseases.

## Description

### Background to the invention

Mesenchymal/stromal stem cells (MSCs) are multipotent progenitor cells, which can be isolated from almost all mesodermal tissue, such as bone marrow (BM), adipose tissue (AT), umbilical cord (UC), among others, with extensive immunosuppressive capabilities. They are able to suppress the pro-inflammatory functions of T cells and promote the generation of regulatory T cells (Tregs), resulting in beneficial effects in a variety of preclinical models of either autoimmune or inflammatory immune-mediated diseases, such as encephalomyelitis, host-versus-graft disease, rheumatoid arthritis, delayed-type hypersensitivity (DTH), type 1 diabetes, celiac disease, lupus, multiple sclerosis, myasthenia gravis, Addison's disease, Crohn's disease and ulcerative colitis among others.

For this reason, MSCs have been widely proposed as the best candidate for the treatment of inflammatory and autoimmune diseases, which has led to more than 90 clinical trials in autoimmune diseases, registered on the NIH site. However, the results of these studies have been variable in preclinical and clinical models, suggesting that the immunoregulatory properties of MSCs require a deeper understanding to optimize their application in the clinic.

In this context, MSCs have been widely shown to respond to several mediators, including the cytokines interferon-gamma (IFNγ) and tumor necrosis factor (TNFα), thereby increasing their immunomodulatory capabilities. Recent studies have revealed that these inflammatory signals are associated with changes in MSC metabolism, characterized by a shift from oxidative phosphorylation to glycolysis. These findings coincide with the results obtained by the inventors, who demonstrated that the metabolic reprogramming of MSCs towards glycolytic metabolism using Oligomycin, a well-recognized inhibitor of ATP synthase, significantly increases their glycolytic bioenergetic status, regardless of the source of MSCs.

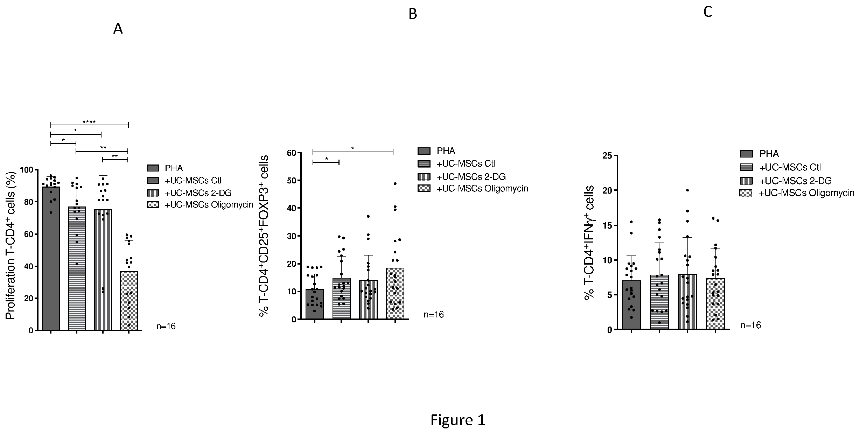

In addition, the results demonstrated that pretreatment of MSCs with Oligomycin, which results in glycolytic MSCs, enhanced their immunosuppressive activity *in vitro* in newly isolated peripheral blood mononuclear cells (PBMCs) activated with phytohemagglutinin (PHAs) to induce T cell activation or proliferation (Figure 1). This effect was also observed on helper T cells (Th) and on CD4-T-cells differentiated into proinflammatory Th1 and Th17 cells.

Likewise, results obtained from *in vivo* experiments, the inventors have determined that, compared to control MSCs, glycolytic MSCs exhibit significantly superior therapeutic efficacy in both the mouse model of delayed-type hypersensitivity (DTH) and graft-versus-host disease (GVHD).

Recent studies have shown that the metabolic status of MSCs is critical to their immunoregulatory and therapeutic efficacy. Based on this, the inventors have hypothesized that the effect of MSCs also depends on their mitochondrial metabolic activity. Previous studies have shown that in cocultures, untreated MSCs can spontaneously transfer mitochondria to CD4-T-cells, inducing a Treg phenotype and improving GVHD progression. In view of these results, the inventors have proposed a strategy of metabolic reprogramming followed by mitochondrial transfer (MT), in which, for the first time, metabolically modified MT mitochondria isolated from MSCs are used to enhance glycolysis, for example, through pretreatment with oligomycin, and transfer them to immune cells, enhancing their activity.

### DESCRIPTION OF THE FIGURES

**Figure 1****. Modification of the expression of the immunosuppressive potential of PBMC T cells from healthy donors incubated with UC-MSC under different metabolic conditions**. PBMCs were isolated from healthy donors, activated with PHA, and cultured alone or in the presence of control UC-MSCs (Ctl), or pretreated with 2-DG or Oligomycin. A) CD4+ T-cells. B) The percentage of cells producing T-CD4+CD25+FOXP3+. C) The percentage of T-CD4+IFNγ+ cells. The results are represented as the mean ± SD considering four independent experiments using at least seven different PBMC donors and four UC-MSC donors. Statistical analyses were performed using a two-way ANOVA test comparing paired samples (*p<0.05; **p<0.01; ***p<0.001, ****p<0.0001).

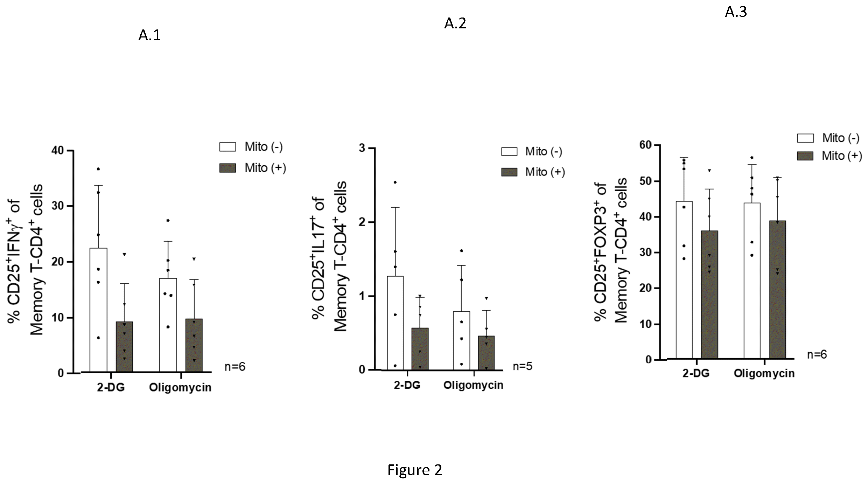

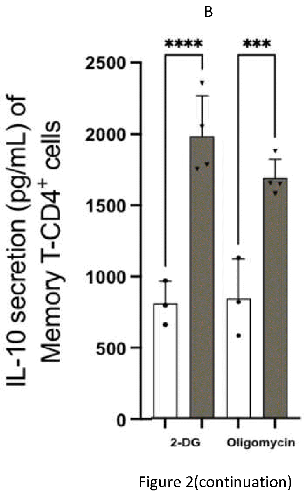

**Figure 2****. Modification of the expression of the immunosuppressive potential of memory T-CD4 cells by artificial mitochondrial transfer (MitoT) of UC-MSC.** Memory T-CD4 cells isolated from healthy donors were incubated with mitochondria isolated from UC-MSCs pretreated with 2-DG (oxidative phosphorylation metabolism) orligomycin (glycolytic metabolism). The expression of different markers between untransformed (Mito -) and transformed controls with mitochondria (Mito +) of both conditions is compared. The percentage of A) cells producing T-CD4+CD25+IFNγ+ (A.1), T-CD4+CD25+IL17+ (A.2) and T-CD4+CD25+FOXP3+ (A.3) is shown. B) It shows the level of the anti-inflammatory cytokine, IL-10. The results are represented as the mean ± SD (standard deviation) of four independent experiments using at least seven different PBMC donors and four UC-MSC donors. Statistical analyses were performed using a two-way ANOVA test comparing paired samples (*p<0.05; **p<0.01; ***p<0.001, ****p<0.0001).

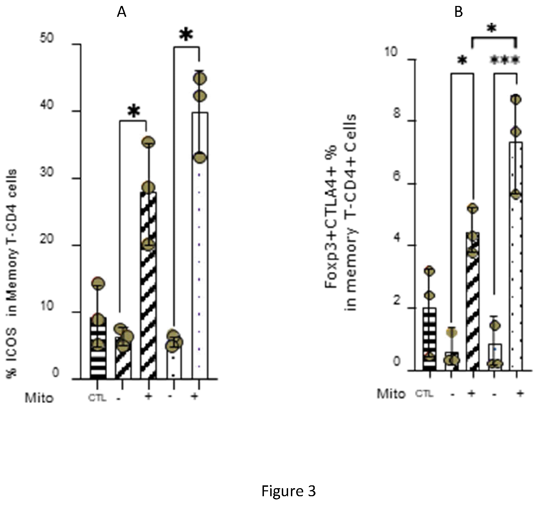

**Figure 3****. Modification of immunosuppressive potential expression of memory T-CD4 cells of patients with RA by artificial mitochondrial transfer (MitoT) from UC-MSC.** Memory T-CD4 cells isolated from donors with rheumatoid arthritis were incubated with mitochondria isolated from UC-MSCs pretreated with 2-DG (oxidative phosphorylation metabolism) represented with diagonal stripes or oligomycin (glycolytic metabolism) represented with a dotted pattern. The control is displayed in horizontal stripes. The expression of different markers between untransformed (Mito -) and transformed controls with mitochondria (Mito +) of both conditions is compared. The percentage of A) percentage of memory CD4 T cells expressing ICOS and B) percentage of memory T-CD4 producing cells expressing FOXP3+ CTLA4+ is shown. The graphs represent the mean ± SD and the statistical analysis using the non-parametric Mann-Whitney test between two parameters (comparison between MITO+ or MITO- or MITO+CTL vs MITO+OLIGO). Each dot represents a different HD with at least 3 different donors.

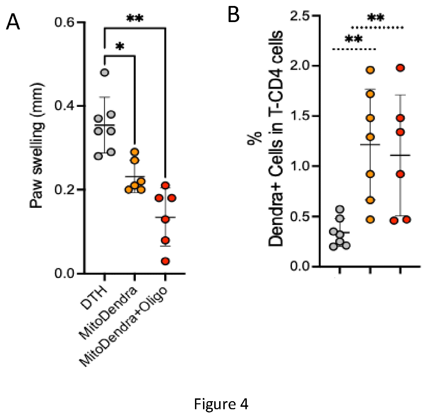

**Figure 4****. Effect of UC-MSC Mitochondria in an in vivo model of hypersensitivity-mediated inflammation.** A) Effect of TCD4 cells from a mouse model of hypersensitivity-mediated inflammation (DTH), those containing MSC mitochondria labeled with dendra fluorophore, and where MSCs had or had not been pretreated with Oligomycin prior to mitochondrial isolation for mitoception. Inflammation of the animal's leg is measured under control and with CD-4 T-cell treatment with dendra-labeled MSC mitochondria (MitoDendra) and with CD-4 T-cell treatment with dendra-labeled MSC mitochondria and pretreated with Oligomycin (MitoDendra+Oligo). It shows that treatment with MSC T-cells and mitochondria significantly decreases inflammation, and that MSC mitochondria pretreated with Oligomycin have an even greater anti-inflammatory effect. B) Graph showing the mitoception of both types of mitochondria in CD4-T-lymphocytes, those obtained from MSCs (MitoDendra) and those obtained from MSCs pretreated with Oligomycin (MitoDendra+Oligo).

### DESCRIPTION OF THE INVENTION

The present invention proposes a method to enhance the anti-inflammatory activity of T cells, by acquiring functional mitochondria isolated or obtained from umbilical cord mesenchymal stem cells (UC-MSCs). Where these UC-MSCs preferentially undergo a modification that induces an increase in their glycolytic metabolism before extracting their mitochondria.

T cells modified with these mitochondria can be reimplanted in the individual with the pathology, where they will contribute to reducing inflammatory processes in diseases such as rheumatoid arthritis (RA) or in delayed hypersensitivity (DTH) pathologies or in graft-versus-host disease (GVHD), as well as in other pathologies related to immune imbalances, and other autoimmune and/or inflammatory diseases such as encephalomyelitis, type 1 diabetes, celiac disease, lupus, multiple sclerosis, myasthenia gravis, Addison's disease, Crohn's disease and ulcerative colitis among others.

The inventors studied the immunoregulatory potential of mitochondria (MTs) obtained from both unmodified UC-MSCs and those with activated glycolytic metabolism. This study sought to determine whether TMs derived from glycolytic MSCs induced greater differentiation of CD4+ T cells into a regulatory phenotype (Treg) and greater repression of Th17 cells (pro-inflammatory phenotype) compared to TMs obtained from unmodified MSCs.

To clearly discriminate the level of TM-dependent immunoregulatory impact without interference from other factors, the inventors used an artificial mitochondrial transfer approach called mitoception, in which purified MTs were incubated with memory T-CD4 cells. Subsequently, they evaluated fenotypic, functional, and metabolic changes of post-mitoception CD4 T-cells.

In the functional assay, the inventors measured the expression of anti-inflammatory cytokines, such as IL-10, in memory T-CD4 cells, analyzing their pro-inflammatory and anti-inflammatory phenotype using fluorescence activated cell sorting (FACS). In addition, to induce glycolytic metabolism in MSCs, Oligomycin was used. Initially, peripheral blood mononuclear cells (PBMCs) from healthy donors were co-cultured with UC-MSC cells pretreated with 2-DG (glycolysis metabolism inhibitor) or oligomycin, compared to both metabolic conditions. The results are shown in Figure 1, and show that pre-treatment with oligomycin increased the immunosuppressive effect of UC-MSCs on PBMC-derived T cells, in terms of reducing the proliferation of the latter. The inventors then isolated mitochondria from UC-MSCs pre-treated or not with 2-DG or Oligomycin, revealing even stronger immunoregulatory results than whole cells, although no significant differences were observed between mitochondria derived from UC-MSCs pre-treated with 2-DG or Oligomycin in samples from healthy donors (Figure 2). TMs from both types of pretreatment decreased the expression of pro-inflammatory markers such as IFNγ, IL-17, and CD25+FOXP3+ and increased the expression of IL-10, an anti-inflammatory cytokine. indicating an increase in the anti-inflammatory activity of T cells.

Preliminary results, obtained in PBMC-derived T cells from healthy donors, indicate an improvement in the anti-inflammatory activity of T cells incubated with mitochondria isolated from UC-MSCs. *A priori*, no significant differences were observed between UC-MSC mitochondria with and without induced glycolytic metabolism. However, when using T cells from donors with inflammatory and autoimmune diseases, such as rheumatoid arthritis, differences were observed: mitochondria derived from glycolytic UC-MSCs showed a superior effect on anti-inflammatory capacity on the T cells of these donors.

This result validates the researchers' initial hypothesis, demonstrating that UC-MSC mitochondria with induced glycolytic metabolism are more effective in enhancing the anti-inflammatory activity of T cells in individuals with inflammatory pathologies, such as rheumatoid arthritis (RA), delayed hypersensitivity (DTH) or graft-versus-host (GVHD); encephalomyelitis, type 1 diabetes, celiac disease, lupus, multiple sclerosis, myasthenia gravis, Addison's disease, Crohn's disease and ulcerative colitis among others.

These results offer a strategy to obtain peripheral blood T cells from individuals with an immune disease, with an inflammatory component, such as RA, DTH or GVHD, and incubate them with mitochondria isolated from UC-MSCs pretreated with oligomycin and thus induce a glycolytic state before mitochondrial extraction. The T cells treated in this way can be reimplanted in the same individual, functioning as an autologous treatment with increased anti-inflammatory capacity, which would help mitigate the symptoms of these diseases (RA, DTH, GVHD, lupus or other diseases with immune imbalance).

Because this procedure uses internally modified T cells, the risks associated with conventional biologic therapies are reduced. Taken together, these results suggest that MSC-derived mitochondria, particularly when metabolically reprogrammed into a glycolytic profile, represent a promising therapeutic approach to modulate proinflammatory memory T-CD4 cells, intervening in processes of immunoregulatory imbalance and autoimmune diseases such as RA, DTH and GVHD encephalomyelitis, type 1 diabetes, celiac disease, lupus, multiple sclerosis, myasthenia gravis, Addison's disease, Crohn's disease and ulcerative colitis among others.

Thus, the invention refers to a method for inducing anti-inflammatory or regulatory T-cell phenotype in a population of T-cells, where this method comprises the following steps:
a. Obtaining a culture of umbilical cord mesenchymal stem cells (UC-MSCs);
b. Extracting mitochondria from cells from the previous culture and resuspending them in a buffer solution at a concentration between 50 and 200 mg of mitochondria per mL;
c. Obtaining the fraction of mononuclear cells from an isolated peripheral blood sample (PBMC), and isolating the memory T-CD4 cells using a kit, and keeping them in culture;
d. Incubating the memory T-CD4 cells obtained from step c) with the mitochondria obtained from step b) and keeping them in culture;
e. Obtaining memory T-CD4 cells that have incorporated the mitochondria of UC-MSCs into their cytoplasm, thus modifying their metabolism towards a regulatory T cell phenotype with anti-inflammatory properties.
f. Resuspending the T cells obtained in a buffer solution to obtain a composition of T cells with an anti-inflammatory or regulatory T cell phenotype.
Where in step b) mitochondria are extracted from an amount of about an amount of around 10⁵ to 10⁷ cells from the MSC culture of step a). And additionally, in step d) the suspension of mitochondria obtained in step b) is carefully applied to the culture of memory T-CD4 cells obtained from step c), and the culture plates are centrifuged at 1500 g for 15 min at 4 °C. Subsequently, in step d) the memory T-CD4 cells already centrifuged with the mitochondria, are incubated for 1 to 4 hours, at 37 °C and then subjected to a second centrifugation at 1500 g for 15 min at 4 °C. Finally, in step d), after the second centrifugation, memory T-CD4 cells incubated with mitochondria are cultured at 37°C for 8 to 14 hours.

In a second realization the invention points to the method already described, where optionally in step a) umbilical cord mesenchymal stem cells (UC-MSC) are pre-treated with oligomycin in a concentration between 0.1 to 10 µg/ml for 12 to 48 hours; and then a wash is carried out with a buffer solution.

In a third embodiment, the invention points to the use of memory T-CD4 cells obtained according to any of the above methods, where these modified cells serve to be incorporated into an injectable solution comprising the cells and a pharmacologically acceptable buffer solution, where the T cells have phenotypic anti-inflammatory or regulatory T cell properties. Where the injectable solution is useful to mitigate the symptoms of a disease in cases of immune imbalances or autoimmune diseases. Specifically, immune or autoimmune imbalance diseases are rheumatoid arthritis (RA), delayed hypersensitivity (DTH) or graft-versus-host disease (GVHD), type 1 diabetes; Celiac disease; Lupus; Multiple sclerosis; Myasthenia gravis; Addison's disease; Crohn's disease; Ulcerative colitis. In a preferred embodiment, the T cells modified by the method of the invention are conveniently injected autologously, into the peripheral blood donor.

It is obvious to the expert in the field that a method that uses living blood cells and mitochondria must always work with appropriate buffers to preserve cellular and mitochondrial integrity. Where the nature of the buffer solution may vary, as it is not relevant in itself. The inventors prefer to use buffered saline solution (PBS) and DMEM medium is preferentially used as culture medium.

The invention will be clearer in the light of the following illustrative examples.

### EXAMPLES.

### Example 1. Effect of UC-MSCs on PBMC T cells derived from healthy donors

First, the inventors conducted a study to establish whether glycolytic metabolism effectively enhances the immunosuppressive potential of UC-MSCs in PBMC-derived T cells from healthy donors.

### 1.1.Obtaining UC-MSC

Umbilical cord-derived mesenchymal stem cells (UC-MSCs) were cultured in DMEM medium supplemented with 10% FBS, Penicillin/Streptomycin, and glutamine (DMEM-10) until 80% confluence was reached when the culture medium was removed and washed once with buffered saline (PBS).

### UC-MSC Oligomycin

For glycolytic stimulation, fresh DMEM-10 was added to the UC-MSCs supplemented with an oligomycin solution containing the A, B, C isomers at a final concentration of 1µg/ml, and incubated for 24 hr. Subsequently, the medium that could contain some traces of oligomycin was removed and the cells were washed with buffered saline (PBS), thus obtaining the UC-MSC oligomycin cells that have an activated glycolytic metabolism.

### UC-MSC 2-DG

For control without glycolytic stimulation, fresh DMEM-10 was added to the UC-MSCs supplemented with a solution of 2-DG at a final concentration of 10 mM, and incubated for 24 hr. Subsequently, the medium containing 2-DG was removed and the cells were washed with buffered saline (PBS), thus obtaining the UC-MSC 2-DG cells that have an inhibited glycolytic metabolism.

### 1.2.Co-culture of UC-MSCs and PBMCs

Peripheral blood mononuclear cells (PBMCs) were isolated from healthy donors and stained with CTV (*CellTrace Violet*). They were also activated with phytohemagglutinin (PHA) (5 µg/mL) for 96 hours, to induce the activation and proliferation of T cells. These PBMCs were cultured alone or co-cultured with control UC MSCs, or pretreated with 2-DG or oligomycin in a PBMC:UC-MSC ratio of 20:1, for 4 days, under usual conditions of human cell growth (37°C, atmosphere with 5% CO₂, humidified). After 96 hours of co-culture, the different phenotypes (pro and anti-inflammatory) of the T cells were evaluated by FACS (fluorescence activated cell classification), the results are shown in Figure 1.

CD4+ T-cells (or T helper) were identified based on CD4 surface marker expression and proliferation was measured as the decrease in CTV fluorescence intensity. The results are shown in (Figure 1 A.) When PBMC was co-incubated with UC-MSC under different conditions, a decrease in the proliferation of these cells was observed compared to the control, which is stronger when using UC-MSC with glycolytic metabolism (Oligomycin).

When the result is broken down, it is observed that the percentage of T-CD4+CD25+FOXP3+ cells, markers of regulatory T cells, is increased with the presence of UC-MSC incubated with Oligomycin (Figure 1 B). On the other hand, the percentage of T-CD4+IFNγ+ cells, markers of Th1 cells, does not show variations in the different conditions (Figure 1 C). Both T cell types were measured by specific intracellular staining to identify IFNγ and FOXP3 in PBMCs. Overall, we can say that co-culturing T cells with UC-MSC slightly increases the percentage of regulatory T cells, which increases when mesenchymal cells have been pretreated with Oligomycin.

### Example 2. Effect of UC-MSC Mitochondria on PBMC-derived T cells from healthy donors

Since an advantage was established in example 1 when incubating with UC-MSC cells with glycolytic metabolism (pretreated with Oligomycin). It was decided to study the effect of the mitochondria of these cells.

### 2.1 Mitochondria Retrieving UC-MSC

The cells obtained in example 1: UC-MSC Oligomycin and UC-MSC 2-DG, in this case mitochondrial donor cells, were labeled with a MT-specific fluorescent probe (*MitoTracker Green*) and then their mitochondria were isolated. The mitochondria were isolated using a commercial mitochondrial isolation kit for cultured cells (Thermo Scientific) following the manufacturer's instructions. The isolated mitochondrial preparations were made using the order of 10⁶ MSCs. A yield of 100 mg of mitochondria per million MSCs was obtained. The isolated mitochondria were resuspended in 1ml of MLR (Mix Lymphocyte Reaction) medium, kept on ice, and immediately used for artificial transfer or Mitoception.

### 2.2 Transformation of T Cells by Mitoception of Isolated Mitochondria.

The transfer of UC-MSC mitochondria thus obtained onto memory T-CD4 cells derived from PBMCs from healthy donors were combined into a ratio of mitochondria from 1 UC-MSC cell to 20 memory T-CD4 cells isolated from PBMC. The mitochondrial receptor cells were kept in an MLR medium and the mitochondrial suspension was slowly added, near the bottom of the well, throughout the culture surface. The culture plates were then centrifuged at 1500 g for 15 min at 4 °C. They were then placed in an incubator at 37 °C and 5% CO₂. The next day, the cells were analyzed using FACS to check for Mitoception. Memory T-CD4 cells were sorted using FACS, and were classified as either *MitoTracker* Green-positive (Mito+) or *MitoTracker-Green* negative (Mito-). Then, the Mito+ and Mito- memory T-CD4 cells were activated with CD3/CD28 and IL-2 microspheres for 96 hours, under usual conditions of human cell growth.

### 2.3 Results

After culture, the different properties of the T-CD4 cells treated by FACS were evaluated. The results are shown in Figure 2. The percentage of cells of T-CD4+CD25+FOXP3+, T-CD4+CD25+IFNγ+ and T-CD4+CD25+IL17+ cells was evaluated. The results are shown in Figure 2-A. Additionally, the level of the anti-inflammatory cytokine, IL-10, was evaluated using the ELISA technique. The results are shown in Figure 2 B.

The results show that in all cases, cells that acquired mitochondria (Mito +), behave in a similar way, regardless of whether the glycolytic metabolism of UC-MSC was activated (Oligomycin) or not (2-DG). In all cases, a small decrease in the subgroups evaluated was observed, being lower for T-CD4+CD25++FOXP3+, markers of regulatory T cells (Figure 2-A.3) compared to T-CD4+CD25+IFNγ+ (Figure 2-A.1) (T h1 cells) and T-CD4+CD25+IL17+, (Figure 2-A.2) (Th17 cells) where a slightly more accentuated decrease was observed. On the contrary, a significant increase in the expression of IL-10, an interleukin with anti-inflammatory activity, is observed in memory T-CD4 cells that acquired mitochondria (Mito+), compared to those that did not acquire mitochondria (Mito-). These results demonstrate a positive effect of UC-MSC-derived mitochondria on memory T-CD4 cells in terms of an increase in their anti-inflammatory capacity, without distinction as to the metabolism of the cell of origin.

### Example 3. Effect of UC-MSC Mitochondria on PBMC-derived T cells from donors with rheumatoid arthritis

Since a treatment such as the one proposed in the present invention will be applied in individuals with some autoimmune and/or inflammatory pathology, the inventors evaluated how the proposed treatment of artificial mitochondrial transfer (mitoception) affects T CD4 cells from patients, specifically in cells obtained from patients with rheumatoid arthritis (RA) of mitochondria obtained from UC-MSCs with different metabolisms.

### 3.1 Transformation of T CD4 Cells from RA Donors by Mitoception of Isolated Mitochondria

The collection of UC-MSC mitochondria pretreated with 2-DG or Oligomycin and the transformation of T CD4 cells with these mitochondria was carried out in the same way as in example 2 (2.1 and 2.2), the only difference being that the donors were individuals with RA. Memory T-CD4 cells were classified by *MitoTracker Green* positive (Mito+) or *MitoTracker Green* negative (Mito-) by *Cell sorting*. Then, the Mito+ and Mito- memory T-CD4 cells were activated with CD3/CD28 and IL-2 microspheres for 96 hours, under usual conditions of human cell growth.

### 3.2 Results

After culture, the different properties of the T-CD4 cells treated by FACS were evaluated. The results are shown in Figure 3.

The percentage of T-CD4+ICOS+, T-CD4+FOXP3+, and T-CD4+CTLA4+ cells was evaluated by intracellular staining to identify FOXP3, ICOS, and CTLA4 in memory T-CD4 cells by FACS. ICOS, FOXP3 and CTLA4 are classic markers expressed in Treg cells, associated with their immunosuppressive activity. The results are shown in Figure 3.

The results show that in all cases, cells that acquired mitochondria (Mito +), show better results in all these parameters of regulatory T cell activity (Treg), such as the expression of ICOS, FOXP3 and CTLA4+. However, mitochondria obtained from UC-MSCs with induced glycolytic metabolism (pretreated with Oligomycin) show a significant increase in the expression of these molecules compared to mitochondria obtained from untreated UC-MSCs (Figure 3.A and 3.B). These results show that the effect of transforming memory T-CD4 cells with mitochondria from UC-MSC is very positive in modifying these T cells of a sick individual, to regulatory T cells, with anti-inflammatory properties. Where the result is even better when using mitochondria derived from UC-MSC with a glycolytic metabolism, that is, pretreated with Oligomycin.

### Example 4. Effect of mitochondria-modified T cells from UC-MSC in an in vivo model of hypersensitivity-mediated inflammation.

To study the effect of modified T cells according to the method of the invention, an in vivo study was carried out using a mouse model of hypersensitivity-mediated inflammation (DTH), where mice are caused inflammation in one of their legs with an allergenic molecule.

### 4.1 Transformation of T CD4 Cells from Murine Donors by Mitoception of Isolated

### Mitochondria

The obtaining of UC-MSC mitochondria pretreated or not with Oligomycin and the transformation of T CD4 cells with these mitochondria was carried out in almost the same way as in example 2 (2.1 and 2.2), the main difference being that the murine model is worked on and that the mitochondria were marked with the dendra fluorophore. We worked with 6 to 7 animals per condition. Figure 4 B) shows that T-CD4 cells mitocept in a similar proportion both mitochondria obtained from UC-MSC with its unmodified metabolism (MitoDendra) and those obtained from MSCs pretreated with Oligomycin (MitoDendra+Oligo). Proving the point that despite mitocepting in a similar percentage, mitochondria obtained from oligomycin-pretreated MSCs have a better decrease in inflammation, as shown in Figure 4A).

Each animal with the DTH model was injected with 200 uL of T cells with the mitochondria incorporated in PBS buffer. And left to act for 12 hours.

### 4.2 Results

The results are shown in Figure 4 A). Inflammation is observed in the control condition and with CD-4 T-cell treatment with dendra-labeled MSC mitochondria (MitoDendra) and with CD-4 T-cell treatment with dendra-labeled MSC mitochondria and pretreated with Oligomycin (MitoDendra+Oligo). It shows that treatment with MSC T-cells and mitochondria significantly decreases inflammation, and that MSC mitochondria pretreated with Oligomycin have an even greater anti-inflammatory effect.

This demonstrates that the method and treatment proposed in the invention allows for modifying T cells towards an anti-inflammatory or regulatory T cell phenotype, which effectively allows for alleviating inflammatory symptoms in vivo, without an increased risk associated with this treatment.

## Claims

1. A method to induce anti-inflammatory phenotype and/or regulatory T-cell phenotype in T-cells **CHARACTERIZED in that** it comprises the following steps:
a. Obtaining a culture of umbilical cord mesenchymal stem cells (UC-MSCs);
b. Extracting mitochondria from cells from the previous culture and resuspending them in a buffer solution at a concentration between 50 and 200 mg of mitochondria per mL;
c. Obtaining the fraction of mononuclear cells from an isolated peripheral blood sample (PBMC), and isolating the memory T-CD4 cells using a kit, and keeping them in culture;
d. Incubating the memory T-CD4 cells obtained from step c) with the mitochondria obtained from step b) and keeping them in culture;
e. Obtaining memory T-CD4 cells that have incorporated the mitochondria of UC-MSCs into their cytoplasm, thus modifying their metabolism towards a regulatory T cell phenotype with anti-inflammatory properties;
f. Resuspending the T cells obtained in a buffer solution to obtain a composition of T cells with an anti-inflammatory or regulatory T cell phenotype.

2. Method according to claim 1 **CHARACTERIZED in that** in step b) mitochondria are extracted from a quantity of about a quantity of around 10⁵ to 10⁷ cells from the MSC culture of step a).

3. Method according to claim 1 **CHARACTERIZED in that** in step d) the suspension of mitochondria obtained in step b) is carefully applied to the culture of memory T-CD4 cells obtained from step c), and the culture plates are centrifuged at 1500 g for 15 min at 4 °C.

4. Method according to claim 3 **CHARACTERIZED in that** in step d) the memory T-CD4 cells already centrifuged with the mitochondria, are incubated for 1 to 4 hours, at 37°C and then subjected to a second centrifugation at 1500 g for 15 min at 4 °C.

5. Method according to claim 4 **CHARACTERIZED in that** at step d) after the second centrifugation of memory T-CD4 cells incubated with mitochondria they are cultured at 37°C for 8 to 14 hours.

6. Method according to claim 1 **CHARACTERIZED in that** optionally in step a) umbilical cord mesenchymal stem cells (UC-MSCs) are pre-treated with oligomycin at a concentration between 0.1 to 10 µg/ml for 12 to 48 hours; and then washed with a buffer solution.

7. Method according to claim 6 **CHARACTERIZED in that** in step b) mitochondria are extracted from a quantity of about a quantity of around 10⁵ to 10⁷ cells from the MSC culture of step a).

8. Method according to claim 6 **CHARACTERIZED in that** in step d) the suspension of mitochondria obtained in step b) is carefully applied to the culture of memory T-CD4 cells obtained in step c), and the culture plates are centrifuged at 1500 g for 15 min at 4 °C.

9. Method in accordance with claim 8 **CHARACTERIZED in that** in step d) memory T-CD4 cells already centrifuged with the mitochondria, are incubated for 1 to 4 hours, at 37°C and then subjected to a second centrifugation at 1500 g for 15 min at 4 °C.

10. Method according to claim 9: **CHARACTERIZED in that** at step d) after the second centrifugation, memory T-CD4 cells cultured with mitochondria are cultured at 37°C for 8 to 14 hours.

11. Use of memory T-CD4 cells obtained in accordance with any of claims 1 through 10 **CHARACTERIZED in that** they serve to be incorporated into an injectable solution comprising the cells and a pharmacologically acceptable buffer solution, where T cells have anti-inflammatory or regulatory T cell phenotypic properties.

12. Use according to claim 11 **CHARACTERIZED in that** the injectable solution is useful in mitigating the symptoms of a disease in cases of immune imbalances or autoimmune diseases.

13. Use according to claim 12 **CHARACTERIZED in that** immune or autoimmune imbalance diseases are rheumatoid arthritis (RA), delayed-type hypersensitivity (DTH) or graft-versus-host disease (GVHD), type 1 diabetes; Celiac disease; Lupus; Multiple sclerosis; Myasthenia gravis; Addison's disease; Crohn's disease; Ulcerative colitis.

14. Use in accordance with claim 13 **CHARACTERIZED in that** the T cells modified by the method of the invention are conveniently injected autologously, into the peripheral blood donor.
